# EUROPEAN PATENT APPLICATION

(11) **EP 3 056 088 A2**
(43) Date of publication of application: **17.08.2016**
(21) Application number: 16165098.1
(22) Date of filing: 13.04.2016
(51) Int. Cl.: A23C 19/06

(54) **CHEESE RIPENING**

(71) Applicant: DSM IP Assets B.V., 6411 TE Heerlen (NL)
(72) Inventor: PRICE, Claire Emile, 6100AA ECHT (NL); MALJAARS, Cornelia Elizabeth Paulina, 6100 AA ECHT (NL); DERKS, Eduard Peter Paul Antoine, 6100 AA ECHT (NL)
(74) Representative: Kuster, Janaart Frans

(57) **Abstract**

The present invention relates to a method for monitoring the ripening process of cheese, which method comprises: (i) providing samples of a ripening cheese over different time points during the ripening process of the cheese; (ii) performing sensory analyses on the samples to determine the intensity of at least one attribute; (iii) quantifying the lactic acid bacteria which are present in the samples; and (iv) correlating the intensity of at least one attribute with the quantified lactic acid bacteria.

## Description

### Field of the invention

The present invention relates to a method for monitoring the ripening process of cheese. According to another aspect, the present invention relates to a method for producing a starter culture for cheese ripening. According to yet another aspect, the present invention relates to Gouda cheese.

### Background of the invention

Cheese ripening is an important part in the manufacture of cheese since it defines the flavor and texture of the cheese, which differentiates the many cheese varieties. Cheese ripening is a process that involves complex and well-balanced reactions between glycolysis, proteolysis and lipolysis of the milk components and further degradation of amino acids. The degradation products of the sugars, milk fat and protein (peptides, free amino acids, di- and mono-glycerides, free fatty acids and amino acid derived compounds such as amines, ketoacids, aldehydes, ketones, alcohols, esters, acids and sulphur compounds) all contribute to the specific taste-, flavour- and texture properties of the cheese in question.

In ripening processes, the formation of taste, flavour and texture is effectuated by endogenous milk enzymes such as plasmin and by exogenous enzymes such as the rennet used for clotting. Furthermore, starter cultures such as lactic acid bacteria used for the initial acidification of the milk, contribute to cheese ripening because a substantial fraction of the bacteria ends up in the curd. Their combined extracellular proteolytic- and intracellular peptidolytic activities and enzymes that further degrade amino acids into the compounds listed above, are capable of breaking down the casein fraction. A source for exogenous enzymes are the dead and / or lysed lactic acid bacteria which release their enzymes into the cheese.

The influence of different species of lactic acid bacteria on cheese ripening and especially flavour formation is poorly understood. An improved insight in the role of different species of lactic acid bacteria is needed for the production of improved starter cultures. Therefore there is a need in the art for methods to monitor the ripening process of cheese.

### Detailed description of the invention

An objective of the present invention, amongst other objectives, is to provide a method to monitor the ripening process of cheese. This objective is met by providing a method according to the appended claim 1. Specifically, this objective is met by providing a method for monitoring the ripening process of cheese, which method comprises:
(i) providing samples of a ripening cheese over different time points during the ripening process of the cheese;
(ii) performing sensory analyses on the samples to determine the intensity of at least one attribute;
(iii) quantifying the lactic acid bacteria which are present in the samples; and
(iv) correlating the intensity of at least one attribute with the quantified lactic acid bacteria.

The inventors of the present invention found that correlating the intensities of flavour attributes with a quantification of lactic acid bacteria provides insight in the role of the lactic acid bacteria in the ripening process. This insight is relevant for the production of starter cultures since now the amounts and species of the lactic acid bacteria in the starter culture can be determined more accurately. Further to the amount and species of lactic acid bacteria, the different strains of the same species can be determined more accurately. For example if found that a certain strain with an early lysis correlates with a desired flavor attribute. Hence, the present method is useful in combining different species, or different strains, of lactic acid bacteria. The present method might also be useful to monitor the performance of strains of lactic acid bacteria in a blended starter culture. For example in a blended starter culture for the production of yogurt or fresh cheeses. The present method is even helpful to monitor the performance of different strains of the same species.

Therefore, the present invention relates to a method for producing a starter culture for cheese ripening, which method comprises:
(i) providing samples of a ripening cheese over different time points during the ripening process of the cheese;
(ii) performing sensory analyses on the samples to determine the intensity of at least one attribute;
(iii) quantifying the lactic acid bacteria which are present in the samples;
(iv) correlating the intensity of at least one attribute with the quantified lactic acid bacteria; and
(v) combining different species of lactic acid bacteria to produce the starter culture.

The ripening process of cheese, as used in the present context, means the phase in cheese production after the initial manufacturing of the cheese is done, wherein the cheese is allowed to mature under controlled conditions. Usually controlled conditions of temperature and relative humidity.

In a preferred embodiment, the present providing samples of a ripening cheese over different time points during the ripening process of the cheese comprises providing samples at time points having an intermediate period of at least 2 weeks. Preferably the intermediate period between providing samples is at least 3 weeks, at least 4 weeks of even at least 5 or 6 weeks. Even more preferably the present providing samples of a ripening cheese over different time points during the ripening process of the cheese comprises providing samples after 6 weeks of ripening, after 12 weeks of ripening, and / or after 24 weeks of ripening. Each time samples are provided one or more samples might be provided. Preferably a sample is an entire cheese which is ripened together with similar cheeses under similar conditions.

The term "starter" or "starter culture" as used herein refers to a culture of one or more food-grade micro-organisms, in particular lactic acid bacteria, which are responsible for the acidification of the milk base. Starter cultures may be fresh (liquid), frozen or freeze-dried. Freeze dried cultures need to be regenerated before use. For the production of a fermented dairy product, the starter is usually added in an amount from 0.01 to 3%, preferably from 0.01 and 0.02 % by weight of the total amount of milk base.

As used herein, the term "lactic acid bacteria" (LAB) or "lactic bacteria" refers to food-grade bacteria producing lactic acid as the major metabolic end-product of carbohydrate fermentation. These bacteria are related by their common metabolic and physiological characteristics and are usually Gram positive, low-GC, acid tolerant, non- sporulating, non-respiring, rod-shaped bacilli or cocci. During the fermentation stage, the consumption of lactose by these bacteria causes the formation of lactic acid, reducing the pH and leading to the formation of a protein coagulum. These bacteria are thus responsible for the acidification of milk and for the texture of the dairy product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp and *Leuconostoc spp,* such as *Lactobacillus delbruekii* subsp. *bulgaricus, Streptococcus salivarius thermophilus, Lactobacillus lactis, Bifidobacterium animalis, Lactococcus lactis, Lactobacillus casei, Lactobacillus plantarum, Lactobacillus helveticus, Lactobacillus acidophilus, Leuconostoc mesenteroides* and *Bifidobacterium breve.*

In a preferred embodiment the present at least one attribute is an odour attribute chosen from the group consisting of intensity, butter, soil, fruity, prickly, sour, sweat, fatty and off odour. More preferably, the present at least one attribute is a flavor attribute chosen from the group consisting of intensity, salt, sour, sweet, dairy, butter, savory, butyric acid, fruity, nutty, off taste, feeling in the mouth, rubbery, hardness, sticky, crumbly, dry, mealy, crystals and fatty.

The present at least one attribute might also be an after taste attribute chosen from the group consisting of intensity, sweet, bitter, soapy and length of aftertaste.

The present at least one attribute might also be an after feel attribute chosen from the group consisting of remains, prickling, fatty and astringent.

In a most preferred embodiment, from all of the above mentioned odour attributes, flavour attributes, after taste attributes and after feel attributes the intensities are determined. It is advantageous to use a broad range of attributes since this improves the correlation with the lactic acid bacteria which are present in the sample. In the event that the present methods are repeated over and over, less attributes might be necessary to use since the knowledge on which attributes are the most correlated with the lactic acid bacteria might increase per time.

In another preferred embodiment, the present performing sensory analyses on the samples to determine the intensity of at least one attribute comprises a descriptive sensory analysis. More preferably a quantitative descriptive analysis method(Stone, H. and Sidel, J.L. "Sensory Evaluation Practises" 3rd edition, 20 2004).

In another preferred embodiment, the present cheese is a cheese which requires a cheese ripening process. More preferably, the present cheese is a hard or semi hard cheese. Most preferably the present cheese is chosen from the group consisting of Gouda, Edam, Parmesan, Brie, Camembert, Stilton, Gorgonzola, Blue cheese, Goat cheese, Swiss, Cheddar, Gruyere, Emmental, Munster, Livanot, Torre de Savoie and Mimolette.

The present quantifying the lactic acid bacteria which are present in the samples is preferably quantifying DNA of the lactic acid bacteria which are present in the samples. The lactic acid bacteria which are present in the samples might be both viable and non viable. Hence, quantifying DNA might be both quantifying DNA of viable lactic acid bacteria and non viable lactic acid bacteria.

In a preferred embodiment, the present quantifying the lactic acid bacteria which are present in the samples comprises a quantitative polymerase chain reaction or qPCR or real time polymerase chain reaction. A qPCR is a highly discrimitatory method to follow the amount of DNA per species present in the sample. More preferably the present qPCR involves the use of a DNA binding dye for selective detection of viable lactic acid bacteria. Such a binding dye binds to the DNA and prohibits the multiplication of the bound DNA in the qPCR. This allows the differentiation between viable and non vialbe cells, or between living and lysed cells. Specifically, total DNA present in the sample can be compared with DNA from living cells and the ratio lysed versus living cells can be determined. An example of a suitable DNA binding dye is propodium monoazide or PMA^{tm}**.** If the qPCR is then carried out after lysis of the lactic acid bacteria cells, the DNA of the viable cells can be selectively quantified. Thus, preferably, the present quantifying the lactic acid bacteria which are present in the samples comprises quantifying damaged and / or dead lactic acid bacteria which are present in the samples and/ or quantifying the viable lactic acid bacteria which are present in the samples. Another advantage of using a DNA binding dye for quantifying DNA of unculurable or non culturable cells is that other (lysed) bacteria than lactic acid bacteria, such as undesired enterococci, can be identified. This is important since these undesired bacteria might still have a negative influence on the cheese ripening process which negative influence can now be explained.

In a further preferred embodiment, the present quantifying the lactic acid bacteria which are present in the samples comprises quantifying the different species of lactic acid bacteria. In another preferred embodiment, the present quantifying the lactic acid bacteria which are present in the samples comprises quantifying the living lactic acid bacteria and/or quantifying the dead lactic acid bacteria.

In another preferred embodiment, the present quantifying the lactic acid bacteria which are present in the samples comprises a quantitative polymerase chain reaction further comprises conversion of the quantitative polymerase chain reaction data to an amount of cells of lactic acid bacteria per gram of cheese. This is advantageous since it provides more insight in the influence of lactic acid bacteria on the cheese ripening process.

In yet another preferred embodiment, the present correlating the intensity of at least one attribute with the quantified lactid acid bacteria is carried out by correlation analysis, preferably a principal component analysis and/or an univariate correlation analysis.

Given the highly advantagous insight in the correlation of attribute with species lactic acid bacteria or dead versus viable lactic acid bacteria, the present combining different species of lactic acid bacteria to produce the starter culture comprises preferably using the correlation of the intensity of at least one attribute with the quantified lactid acid bacteria to determine the amount of species of lactic acid bacteria in the starter culture.

In a further preferred embodiment, the present lactic acid bacteria are chosen from the group consisting of *Streptococcus thermophilus, Lactococcus lactis* spp. *cremoris, Lactococcus lactis* spp. *lactis, Lactobacillus casei, Lactobacillus helveticus, Lactobacillus rhamnosus* and *Leuconostoc* species.

According to another aspect, the present invention relates to Gouda cheese comprising an amount of *Streptococcus thermophilus* cells of more than 8.32 * 10⁸cells / gram cheese after 6 weeks of ripening, an amount of *Streptococcus thermophilus* cells of more than 4.03 * 10⁸ cells / gram cheese after 12 weeks of ripening and / or an amount of *Streptococcus thermophilus* cells of more than 8.49 * 10⁸cells / gram cheese after 24 weeks of ripening.

Further, the present invention relates to Gouda cheese comprising an amount of *Streptococcus thermophilus* cells of more than 1.90 * 10⁹cells / gram cheese after 6 weeks of ripening, an amount of *Streptococcus thermophilus* cells of more than 1.80 * 10⁹ cells / gram cheese after 12 weeks of ripening and / or an amount of *Streptococcus thermophilus* cells of more than 1.90 * 10⁹ cells / gram cheese after 24 weeks of ripening.

### Figure legends

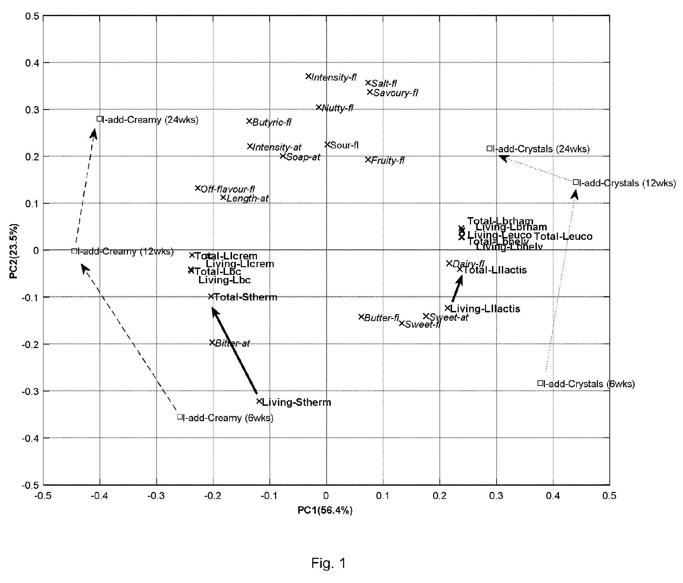

Figure 1: Combined PCA biplot for qPCR (log10(species counts)) and QDA flavor data. Both datasets were scaled to unit length. The bacterial names are abbreviated for visualisation purposes: *S*. *thermophilus* (Stherm), *Lb. rhamonosus* (Lbrham), *Lb. helveticus* (Lbhelv), *Leuconostoc* species (Leuco), *Lb. casei* (Lbc), *L. lactis* ssp. *lactis* (Lllactis) and *L. lactis* ssp. *cremoris* (Llcrem). The prefix before the species name indicates whether the values are for the total population quantitated or only the living part.

### Example

Use of qPCR (Quantitative Polymerase Chain Reaction) to assign flavor development in ageing Gouda cheese to bacterial populations. Define which bacterial species are related to which (sensory specific) flavor developments in ageing Gouda cheese and in which frequencies and which analytical and sensory methods were used.

### 1. Materials and methods

### 1.1. Cheese make

Full fat Gouda was made as follows: Raw milk (standardized but not homogenized) was pasteurized at 73°C for 15 seconds. The milk leaves the pasteurizer at a temperature of 31-32°C and during the filling of the 200 liter vats, annatto, calcium chloride and nitric acid were added sequentially. The starter culture was added at the required dosage and 20 minutes after this, rennet was added at the required dosage. When the gel had reached the required firmness, it was cut into cubes of approximately 1 cm³. The cutting process took 20 minutes. After this, 40% of the whey was removed and replaced by 30% warm water addition, based on the drained volume. The temperature of the water was set at 43°C to obtain a temperature of 33.5-34°C for the total curd/whey mixture. The curd/whey mixture was then stirred for one hour, after which, the curd was collected and rested in the pre pressing frame. After a resting time of 15 minutes, the curd was divided into 4 even squares and lifted into the moulds and rested for another 15 minutes after which the pressing starts. This consisted of 3 different slots of 15, 45 and 30 minutes at 0.7, 1.2 and 1.7 bar respectively.

A pH range of pH 5,4-5,6 in five hours after starter culture addition is required for the cheeses to proceed to the brine (21% Baume, pH 5.0) for 24 hours. After brining, the cheeses were dried, coated and moved to the ripening room (13°C, 88% realtive humidity). For the first two weeks, the cheeses were turned and coated three times a week. After this time, they were turned twice a week and coated once a month. Cheeses were removed from the ripening cell for analysis after 6, 12 and 24 weeks of ripening.

Gouda cheese was made using frozen direct vat set culture from DSM Food Specialties. DELVO®CHEESE MT82A was used as a starter culture together with the adjunct cultures DELVO®CHEESE I-ADD CRYSTALS DSF or DELVO®CHEESE I-ADD CREAMY DSF(DSM Food Specialties). The coagulant MAXIREN® 600 was used (DSM Food Specialties).

### 1.2. Sensory

### 1.2.1. Sample preparation

Prior to the panel session, the Gouda cheese was cut into triangular pieces. As the Gouda cheese has a salt gradient a cutting protocol has to be followed. The cheese wheels were cut in half and one of the halves was cut in two pieces. These bigger pieces were cut in three even wedges. About 1,5 cm of the rind of the cheese was removed and from this wedge about 6-7 triangular pieces were cut. Hereafter the samples were placed in an expanded polystyrene box to prevent them from warming up. The products were placed in a polystyrene sample holder and given to the panel members. The panel members were instructed to break the cheese in the middle of the triangular piece and were instructed only to consume the area where the cheese was broken.

### 1.2.2. Data acquisition

### Descriptive sensory analysis was done by using the Quantitative Descriptive

Analysis Method (Stone, H. and Sidel, J.L. "Sensory Evaluation Practises" 3rd edition, 20 2004). First, the panelists developed a list of attributes including definitions by means of evaluating a wide variety of references and a wide array of cheeses. Secondly, training sessions were organized to enable panelists to learn to consistently differentiate and replicate the cheese samples. During the QDA measurement the intensities of the selected attributes were obtained per product by the FIZZ (Biosystems; France) sensory data acquisition system, using unstructured line scales ranging from 0 - 100. The products were evaluated twice by 10 - 13 panelists. As the aim of the experiment was to measure the differences between the products, the products were given at random in order to avoid sequence effects. The products were given one-by-one to the panelists. Statistical analysis of the data was done by analysis of variance with Fisher's least significant difference (LSD) as a post hoc test (SenPaq) and modelled using Principle Component 30 analysis (PCA) (SenPaq).

### 1.2.3. Attributes

Below the attributes and the definitions used during assessing the Gouda cheese.

### Odour

**Intensity** which is the general and overall odour intensity.
**Butter** which is the odour of butter.
**Soil** which is the intensity of an earthy odour (e.g. mushroom, musty).
**Fruity** which is the degree of a fruity odour that is present in the product (e.g apple, pineapple).
**Prickly** which is the intensity of a prickly odour (e.g. sharp, spicy, piquant).
**Sour** which is the intensity of a sour odour, like that of buttermilk.
**Sweat** which is the intensity of a sweaty, whey-like, odour.
**Fatty** which is the intensity of a fatty, creamy odour.
**Off odour** which is a group of odours that cannot be assessed by one of the previous odour attributes.

### Flavor

**Intensity** which is the general and overall flavor intensity
**Salt** which is the degree of salty taste.
**Sour** which is the degree of a sour taste.
**Sweet** which is the degree of a sweet taste.
**Dairy** which is the intensity of a dairy taste (e.g. milk, yoghurt, crème fraîche)
**Butter** which is the intensity of a real butter taste
**Savory** which is the intensity of a broth and meaty taste
**Butyric acid** which is the intensity of a butyric acid flavour (e.g. baby vomit, Swiss powdered cheese). This flavor is not related to butter.
**Fruity** which is the intensity of a fruity taste (e.g. apple, pineapple)
Nuttywhich is the intensity of a nutty taste (e.g. almond, peanut, rucola lettuce)
**Off taste** which is a group of flavors that cannot be assessed by one of the previous flavor attributes.
**Feeling in the mouth -** assessment of the mouthfeel when the product is inside the mouth
**Rubbery** which is the degree in which the product has an elastic character (rubber,
pliable, elastic) of the product. This attribute has to be assessed by hand.
**Hardness** which is the degree of firmness of the cheese (soft, hard).
**Sticky** which is the degree of the stickiness of the product to the palate and teeth during mastication.
**Crumbly** which is the degree of the formation of crumbs during chewing.
**Dry/mealy** which is the degree of a dry/mealy feeling in the mouth during mastication of the product.
**Crystals** which is the degree of crystals that are present in the cheese.
**Fatty** which is the degree of the formation of a fatty (greasy) layer in the mouth (forms a film).

### After taste (to be judged after swallowing of the product)

**Intensity** which is the total intensity of after taste.
**Sweet** which is the intensity of a sweet after taste.
**Bitter** which is the intensity of a bitter after taste.
**Soapy** which is the intensity of long fatty acids (e.g. soap, metallic).
**Lenght after taste** which is the degree in which a products lingers in time in the mouth.

### After feel (to be judged after swallowing of the product)

**Left overs** which is the amount of product that remains in the mouth after swallowing.
**Tingeling** which is the amount of a prickly sensation that is left behind in the mouth (e.g. salt, carbon dioxide-like).
**Fatty** which is the extent of the full, fatty feeling that is present after swallowing.
**Astringent** which is the degree of an astringent (rough, tart) feeling in the mouth and resembles the feeling after eating walnuts or drinking red wine

### 1.3. qPCR

### Cell purification from Cheese matrix

For cheese samples, DNA extraction was performed according to van Hoorde *et al*., 2008. Briefly, 0,2 g of cheese was suspended in tri-sodium citrate solution (pH 8) to which zirconium beads (1,0 mm) were added. Sequential bead beating and washing steps were performed to remove fat from the sample.

### PMA treatment of cheese samples

1 µl PMA (Stock 10 mM) was added to the cell suspension and mixed. The tubes were incubated in the dark for 5 minutes, with occasional mixing. The tubes were then exposed to blue light on the PMA-PhotoLED device for 15 minutes to cross link the PMA to the DNA.

### DNA extraction

The resulting bacterial cell pellets were incubated for 1 hour @ 37°C with lysozyme and Rnase to improve cell lysis. The DNA extraction was subsequently performed with DNA Masterpure Kit (Epicentre) according to the manufacturer's instructions.

### Primer design

Primers were, where possible, retrieved from the public literature database and validated using type strains.

**Table 1. Primers used**

| **Species/strain** | **Reference** | **Gene** | **Fwd** | **Rev** |
|---|---|---|---|---|
| *L. lactis* ssp. | | | TCCTGATTCACAGTAA ATCCT (SEQ ID No 1) | GCGCACTTGAA TCAGTTTCAA |
| *cremoris* | [1] | *yueF* | | (SEQ ID No 2) |
| | | | TCCTGATTCACAGTAA ATCCT | ATGCCCTCGAA TCTGTTTCAA |
| *L. lactis* ssp. *lactis* | [1] | *yueF* | (SEQ ID No 3) | (SEQ ID No 4) |
| | | | CACCGAGATTCAACAT GG | CGATGCGAATT TCTTTTTC |
| *Lb. casei* | [2] | 16S-23S intergenic | (SEQ ID No 5) | (SEQ ID No 6) |
| | | | ACAAGCTATGAATTCA CTTGC | |
| | [3] | MGB probe | (SEQ ID No 7) | |
| | | | TTATTTGAAAGGGGCA ATTGCT | GTGAACTTTCC ACTCTCACAC |
| *S*. *thermophilus* | [4] | 16S-23S intergenic | (SEQ ID No 8) | (SEQ ID No 9) |
| | | | TTGATGGTGAAGACTT GCTTAGAA | AGGTCCCACTG GTTCGGTCTC |
| *Lb. helveticus* | This study | | (SEQ ID No 10) | (SEQ ID No 11) |
| | | | CCGATGAACAGGCTCA GTGA | CATGTTGTGCG CTTGGAAAA |
| *Lb. rhamnosus* | This study | | (SEQ ID No 12) | (SEQ ID No 13) |
| | | MGB probe | TTGCACTTGATTGTTT CG | |
| | | | (SEQ ID No 14) | |
| | | | CAGGTCTTGACATCCT TTGAAG | CGCTCGTTGCG GGAC |
| *Leuconostoc* | [5] | 16s rRNA | (SEQ ID No 15) | (SEQ ID No 16) |

**Table 2. Annealing temperatures for each primer set used**

| **Species/strain** | **Ta (°C)** |
|---|---|
| *L. lactis* ssp. *cremoris* | 60 |
| *L. lactis* ssp. *lactis* | 60 |
| *Lb. casei* | 60 |
| *S. thermophilus* | 60 |
| *Lb. helveticus* | 60 |
| *Lb. rhamnosus* | 60 |
| *Leuconostoc* species | 60 |

### Reaction conditions

The qPCR reactions were performed using the Biorad CFX96 Touch™ Real-Time PCR Detection System, as per manufacturer's instructions. The annealing temperatures (Ta) for each primer set are given in Table 2. The iQ Multiplex Powermix or iQ™ SYBR® Green Supermix (both Biorad) were used where applicable.

### Correlation qPCR with cell count

Laboratory cultures of type strains were used to generate calibration curves in order to convert the qPCR data to cells/gram of cheese.

### 1.4. Correlations

### 1.5. Correlation study of sensory and bacterial profiles in ageing Gouda Cheese

QDA is a descriptive sensory profiling technique which relies on statistical analysis to assign sensory intensity scores to their causes (products, panelists, designed experimental conditions) [6,7,8,9]. Since the product evaluations are carried out by-design [10] it becomes possible to subtract panelist variance and experimental condition effects (serving orders, carry-over) from the data. QDA panelists were selected from a large pool of candidates (∼10 times the size of the panel) to assemble a panel with proven sensory qualities [6] with respect to sensitivity for odour and taste.

Principal Component Analysis (PCA) [10,11] was used to study the correlation between the log-10 transformed bacterial counts data and the sensory intensity scores for the attributes mentioned above. PCA is a technique to visualize correlations (qPCR versus sensory attributes) and to summarize differences between the cheeses. The log10 transformation [13] was applied to the bacterial counts data to limit unwanted numerical effects from large count numbers (for example to suppress increased residual errors for large count numbers the PCA-model). Both the qPCR and QDA data were normalized to unit length (i.e. subtract mean and divide by the standard deviation) to bring both the qPCR and sensory data on a comparable range, for the purpose of interpretation.

### 2. Results

### 2.1. Sensory

**Odour**

| Names | Ripening time(weeks) | Intensity-od | Butter-od | Mustyod | Butyric -od | Fruityod | Prickling -od | Sourod | Sweatyod | Fattyod | Off-odourod |
|---|---|---|---|---|---|---|---|---|---|---|---|
| I-add-Crystals | 6 | 44,556 | 36,082 | 11,2 | 9,77 | 8,321 | 23,687 | 36,179 | 27,153 | 35,515 | 4,85 |
| I-add-Crystals | 12 | 42,854 | 32,071 | 9,24 | 11,436 | 6,832 | 18,049 | 31,143 | 24,933 | 33,596 | 5,616 |
| I-add-Crystals | 24 | 44,886 | 31,106 | 14,391 | 12,675 | 11,209 | 22,971 | 34,413 | 28,011 | 30,451 | 4,233 |
| I-add-Creamy | 6 | 45,42 | 32,127 | 13,881 | 11,497 | 9,412 | 27,596 | 34,134 | 25,017 | 34,606 | 4,168 |
| I-add-Creamy | 12 | 47,215 | 24,714 | 12,593 | 12,218 | 6,846 | 22,724 | 34,372 | 24,085 | 30,801 | 5,799 |
| I-add-Creamy | 24 | 50,741 | 25,648 | 17,445 | 14,506 | 12,631 | 23,865 | 34,237 | 26,894 | 31,827 | 10,054 |

**Flavor**

| Names | Ripening time(weeks) | Intensity-fl | Salt-fl | Sour-fl | Sweet-fl | Dairy-fl | Butter-fl | Savoury-fl | Butyric-fl | Fruity-fl | Nutty-fl |
|---|---|---|---|---|---|---|---|---|---|---|---|
| I-add-Crystals | 6 | 45,482 | 35,855 | 40,822 | 32,661 | 33,006 | 34,942 | 20,472 | 10,125 | 6,35 | 9,977 |
| I-add-Crystals | 12 | 51,665 | 45,784 | 40,82 | 29,987 | 32,583 | 30,329 | 29,526 | 10,91 | 8,694 | 12,1 |
| I-add-Crystals | 24 | 51,63 | 43,938 | 41,135 | 34,451 | 33,919 | 30,779 | 34,101 | 10,178 | 7,568 | 13,388 |
| I-add-Creamy | 6 | 44,663 | 31,991 | 38,685 | 33,479 | 31,96 | 31,987 | 18,154 | 9,898 | 7,259 | 11,25 |
| I-add-Creamy | 12 | 50,717 | 38,537 | 37,829 | 28,551 | 29,189 | 28,806 | 24,33 | 11,257 | 5,235 | 11,139 |
| I-add-Creamy | 24 | 53,877 | 43,097 | 45,769 | 30,32 | 30,931 | 33 | 29,292 | 11,725 | 8,087 | 13,538 |

| Names | Ripening time(weeks) | Off-flavour-fl | Stickiness-mf | Grainy-mf | Creamy-mf | Sharp-mf | Mealy/dry-mf | Crystals-mf | Fatty-mf | Rubbery-mf | Hardness-mf |
|---|---|---|---|---|---|---|---|---|---|---|---|
| I-add-Crystals | 6 | 9,748 | 49,052 | 12,809 | 43,401 | 11,067 | 15,16 | 1,854 | 49,379 | 41,223 | 14,265 |
| I-add-Crystals | 12 | 11,906 | 45,885 | 6,438 | 38,38 | 19,442 | 17,839 | 3,713 | 43,771 | 35,783 | 25,754 |
| I-add-Crystals | 24 | 10,954 | 42,512 | 11,95 | 35,895 | 16,97 | 23,095 | 1,76 | 38,768 | 37,085 | 33,791 |
| I-add-Creamy | 6 | 13,294 | 37,642 | 18,127 | 33,72 | 13,294 | 23,479 | 1,763 | 41,924 | 43,268 | 24,129 |
| I-add-Creamy | 12 | 15,863 | 41,957 | 6,464 | 31,473 | 17,682 | 19,491 | 3,736 | 40,129 | 38,086 | 33,598 |
| I-add-Creamy | 24 | 16,485 | 45,491 | 9,784 | 33,662 | 18,303 | 23,206 | 2,035 | 43,775 | 28,818 | 54,361 |

**After taste (to be judged after swallowing of the product)**

| Names | Ripening time(weeks) | Intensity-at | Sweet-at | Bitter-at | Soap-at | Length-at |
|---|---|---|---|---|---|---|
| I-add-Crystals | 6 | 42,615 | 28,451 | 34,876 | 12,474 | 47,189 |
| I-add-Crystals | 12 | 45,425 | 24,032 | 30,02 | 13,909 | 47,095 |
| I-add-Crystals | 24 | 43,305 | 28,827 | 29,473 | 15,742 | 45,484 |
| I-add-Creamy | 6 | 42,069 | 25,769 | 39,285 | 14,656 | 46,916 |
| I-add-Creamy | 12 | 48,452 | 23,72 | 37,78 | 14,407 | 48,337 |
| I-add-Creamy | 24 | 46,288 | 24,157 | 36,881 | 15,433 | 49,937 |

**After feel (to be judged after swallowing of the product)**

| Names | Ripening time(weeks) | Left overs -af | Tingeling -af | Fatty-af | Astringent-af |
|---|---|---|---|---|---|
| I-add-Crystals | 6 | 20,383 | 15,117 | 33,071 | 25,448 |
| I-add-Crystals | 12 | 26,415 | 18,739 | 35,234 | 28,485 |
| I-add-Crystals | 24 | 19,442 | 13,456 | 28,15 | 19,43 |
| I-add-Creamy | 6 | 22,974 | 17,344 | 30,525 | 25,402 |
| I-add-Creamy | 12 | 26,366 | 20,988 | 34,08 | 27,778 |
| I-add-Creamy | 24 | 23,107 | 15,369 | 29,31 | 23,663 |

### 2.2. qPCR

Two types of data were generated i.e. total count which included all DNA present in the cells and a living count which was determined after PMA was added to the sample, thus excluding and dead/damaged cells from the analyses. The average values from at least 2 replicates are given in Tables 3 and 4.

**Table 3. Bacterial counts based on qPCR for cheeses made with I-Add Crystals**

| **I add crystals** | | ***S*. *thermophilus*** | ***L. lactis* ssp *cremoris*** | ***L. lactis ssp lactis*** | ***Lb. rhamnosus*** | ***Lb. helveticus*** | ***Leuconos toc*** |
|---|---|---|---|---|---|---|---|
| **Ripening time** | | **cells/g** | **cells/g** | **cells/g** | **cells/g** | **cells/g** | **cells/g** |
| **6 weeks** | Total | 8,32E+08 | 1,01E+08 | 4,90E+08 | 6,83E+08 | 6,95E+07 | 7,11E+06 |
| | Living | 9,39E+08 | 1,00E+08 | 3,87E+08 | 8,60E+08 | 2,75E+07 | 6,38E+06 |
| **12 weeks** | Total | 4,03E+08 | 1,63E+08 | 1,75E+08 | 5,31 E+08 | 2,02E+07 | 5,79E+06 |
| | Living | 5,41 E+08 | 2,84E+08 | 3,46E+08 | 1,64E+09 | 4,97E+06 | 6,29E+06 |
| **24 weeks** | Total | 8,49E+08 | 8,28E+07 | 3,06E+08 | 2,23E+09 | 1,59E+07 | 1,03E+07 |
| | Living | 5,59E+08 | 4,16E+07 | 7,24E+07 | 2,52E+09 | 1,80E+07 | 9,29E+06 |

**Table 4. Bacterial counts based on qPCR for cheeses made with I-Add Creamy**

| **I add creamy** | | ***S. thermophilus*** | ***Lb. casei*** | ***L. lactis* ssp *cremoris*** | ***L. lactis* ssp *lactis*** |
|---|---|---|---|---|---|
| **Ripening time** | | **cells/g** | **cells/g** | **cells/g** | **cells/g** |
| 0 weeks | Total | 7,20E+08 | 3,50E+08 | 6,90E+08 | 4,90E+08 |
| | Living | 1,36E+08 | 4,07E+07 | 7,70E+07 | 2,20E+08 |
| 6 weeks | Total | 1,90E+09 | 1,80E+08 | 1,05E+09 | 5,30E+07 |
| | Living | 1,83E+09 | 4,40E+07 | 6,77E+08 | 2,80E+07 |
| 12 weeks | Total | 1,80E+09 | 1,40E+08 | 8,40E+08 | 2,90E+07 |
| | Living | 1,10E+09 | 6,40E+07 | 4,00E+08 | 1,65E+06 |
| 24 weeks | Total | 1,90E+09 | 1,20E+08 | 1,30E+09 | 4,50E+07 |
| | Living | 6,40E+08 | 3,70E+07 | 6,70E+08 | 4,50E+05 |

### 2.3. Principal Component Analysis (PCA)

Figure 1 shows a PCA-biplot of the combined datasets (qPCR and QDA). The data were first autoscaled so that they both explain an equal amount of variance.

PCA computes principal components in such a way that maximal variance in the data is explained preferably by the fewest number of components. Each component can be considered as a linear combination of each column of the combined data matrix with the restriction that successive components should be independent [12]. This is convenient for visualization (to enable a simple orthogonal biplot-scatterplot like in Figure 1) and interpretation (each component describes a specific part of the variance in the data).

PCA can be computed in many software packages (SAS, MiniTab, R,MATLAB). In this study MATLAB® was used. MATLAB® is a high-level language and interactive environment for numerical computation, visualization, and programming [14]. There are functions available for PCA in various toolboxes but can also be computed via a straightforward singular value decomposition (SVD [12]) to extract the eigenvectors (principal components) from the combined data.

### The PCA-biplot 1- shows two things simultaneously:

1. the PCA scores, which represent the positions of the cheese types (I-ADD CREAMY and I-ADD CRYSTALS at 6, 12 and 24 weeks ripening time) in the combined qPCR and QDA data space;
2: the PCA loadings, which represent the contribution of one of the data vectors (either qPCR counts or sensory attributes) to that specific component. In addition the correlations between the loadings of the qPCR-data and QDA-data and PCA-scores (cheese cultures) can be interpreted. Interpretation support text is provided below.

### Observations from Figure 1:

On the left handside, I-ADD CREAMY is shown for 6, 12 and 24 weeks ripening time. It can be observed that the effect of ripening goes in the upper vertical direction (see dashed arrows), which is in the same direction as most of the flavor intensity attributes except for the *bitter-*at*.* Apparently, this product become less bitter in time and move towards increased intensity scores for most flavor attributes in the upper vertical direction.

On the right handside, I-ADD CRYSTALS is shown for for 6, 12 and 24 weeks ripening time. Here it can be observed that the position of week 6 points in the direction of butter-fl, sweet-fl, sweet-at, dairy-fl and moves in the upper vertical direction during 12 weeks ageing, which is in the same direction as most of the flavor intensity attributes.

Most importantly, it can also be observed that specific species correlate with the sensory data. The trajectory on the left handside for I-ADD CREAMY is close to the development of *S*. *thermophilus* (Stherm), predominantly during within the first 12 weeks ripening time. The largest change is observed in this time frame. On the right hand-side the sensory changes are mostly driven by *L. lactis* ssp. *lactis* (Lllactic) (decline of living species).

For both concepts (I-ADD CRYSTALS and I-ADD CREAMY) show the largest difference between living and total between weeks 6 and 12 whereas there is still a growing flavor intensity going on between 12 and 24 weeks ripening. This could be explained by the persistance of enzyme activity in the cheese matrix even after cell death [14]. Hence the combined use of qPCR and QDA data provides evidence for the existence different metabolic pathways during ageing.

### 2.4. Correlation analysis

The univariate (Pearson) correlation coefficients are also displayed in Table1 for all sensory attributes x species counts. Significant correlations (p(Ho)<0.05) are highlighted in bold text. The table provides additional information to be used together with the PCA-biplot and is provided for convenience.

The Pearson correlation of two variables is always between -1 and 1 (negative versus positive correlation). Small correlations (near zero) indicate that there is no (proven) correlation between the two variables. The statistical significance of a correlation can be assessed with a t-test (depending on the correlation and sample size) or by a permutation test. In this report, a t-test was computed via the cor-function in Matlab. Significant correlations (p(Ho)<0.05) are shown in bold in the Table.

For example, the most (significant) correlations can be found for bitter-at and dairy-fl. For dairy flavor, the total population of *L. lactococcus* spp. *lactis , Lb. rhamnosus, Lb. helveticus* and *Leuconostoc* correlate with this specific attribute, and specifically, the living part of the *Lb*. *rhamnosus, Lb. helveticus* and *Leuconostoc* populations. The same group have a negative correlation with bitterness i.e. decreasing bitterness with increasing living cells of *Lb. rhamnosus, Lb. helveticus* and *Leuconostoc* while a decrease in *S*. *thermophilus* numbers correlates with decreasing bitterness.

Hence, the above information can be used to combine amounts of different lactic acid bacteria for generating a cheese with the desired flavor characteristics.

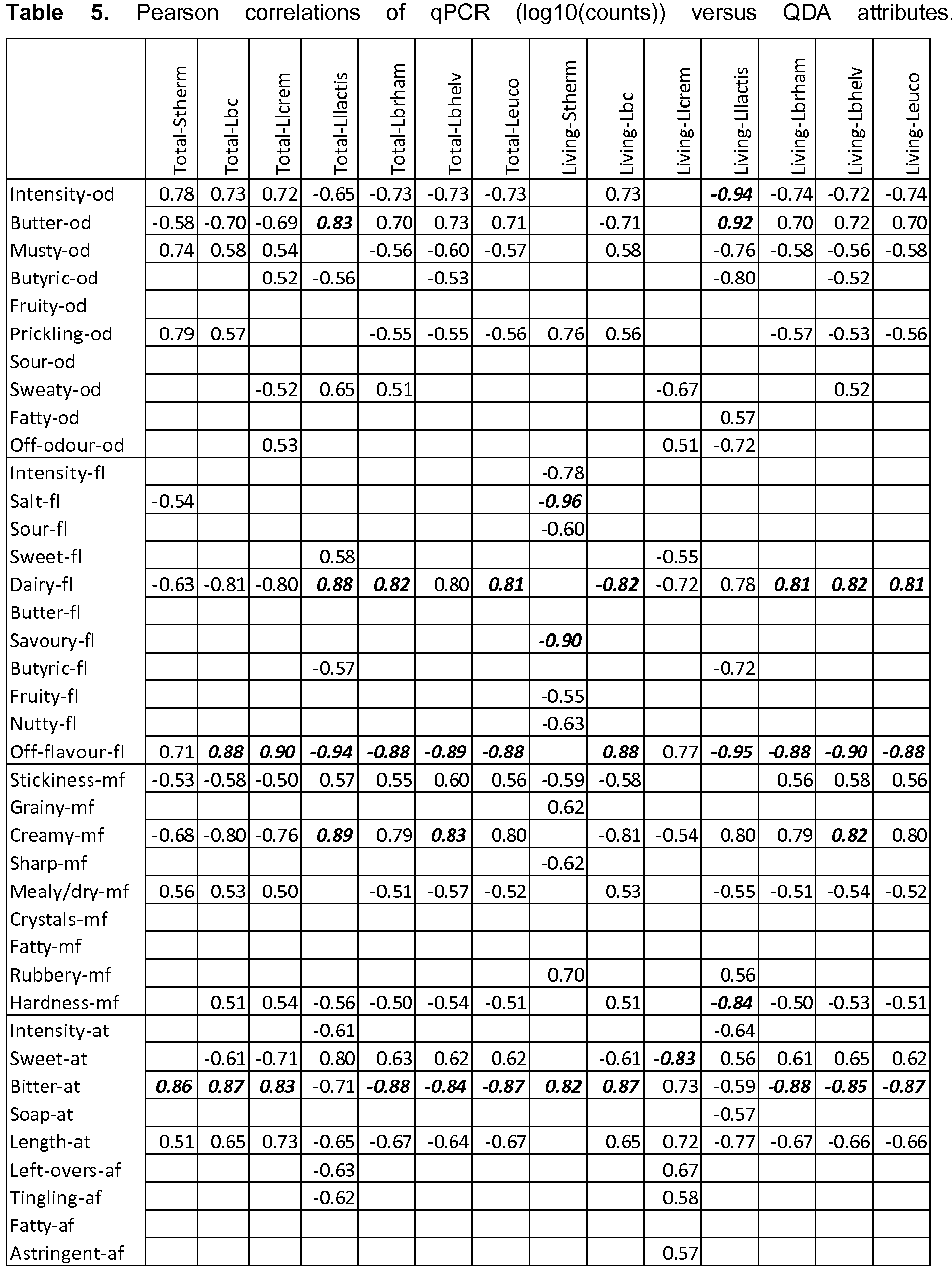

### References

1. P. Khemariya, S. Singha, G. Nathb and A.K. Gulatib. 2013. Subspecies-Specific Nested PCR Assay for Detection of Lactococcus lactis spp. lactis and spp. cremoris. Food Biotechnology. (27)3: 222-234.
2. A.Tilsala-Timisjarvi and T.Alatossava. 1997. Development of oligonucleotide primers from the 16S-23s rRNA intergenic sequences for identifying different dairy and probiotic lactic acid bacteria by PCR. International Journal of Food Microbiology 35: 49-56.
3. M. Haarman and J. Knol. 2006. Quantitative Real-Time PCR Analysis of Fecal Lactobacillus Species in Infants Receiving a Prebiotic Infant Formula. Appl Environ Microbiol. 72(4): 2359-2365.
4. J.P. Furet, P. Quénée, P. Tailliez. 2004. Molecular quantification of lactic acid bacteria in fermented milk products using real-time quantitative PCR. International Journal of Food Microbiology 97:197- 207.
5. U. Friedrich and J.Lenke. 2006. Improved Enumeration of Lactic Acid Bacteria in Mesophilic Dairy Starter Cultures by Using Multiplex Quantitative Real-Time PCR and Flow Cytometry-Fluorescence In Situ Hybridization. Applied and Environmental Microbiology. 72 (6): 4163-4171.
6. ISO 8586-2:1994 Sensory analysis - General guidance for the selection, training and monitoring of assessors.
7. H. Stone and J. Sidel. 1992. Sensory evaluation practices, 2nd edition, Orlando. Academic Press.
8. H. Stone, J. Sidel, S. Oliver, A. Woolsey, R. Singleton. 1972. Sensory evaluation by quantitative descriptive analysis. Food Technology, 28:11, 24-34.
9. M. Meilgaard, G. Civille, B. Carr. 2007. Sensory Evaluation Techniques, CRC Press.
10. W. Cochran, G Cox, 1957, Experimental designs, 2nd edition, Wiley.
11. J. Jolliffe. 2002. Principal Compoent Analysis, 2nd edition, Springer-Verlag.
12. E. Jackson. 2003, A users guide to Principal Components,Wiley-Interscience.
13. G. Box, D. Cox,(1964). An analysis of transformations. Journal of the Royal Statistical Society, Series B 26 (2): 211-252.
14. V. L. Crow, T. Coolbear, P. K. Gopal, F. G. Martley, L. L. McKay and H. Riepeb. 1995. The Role of Autolysis of Lactic Acid Bacteria in the Ripening of Cheese. International Dairy Journal. 5:855-875.

## Claims

1. Method for monitoring the ripening process of cheese, which method
comprises:
(i) providing samples of a ripening cheese over different time points during the ripening process of the cheese;
(ii) performing sensory analyses on the samples to determine the intensity of at least one attribute;
(iii) quantifying the lactic acid bacteria which are present in the samples; and
(iv) correlating the intensity of at least one attribute with the quantified lactic acid bacteria.

2. Method for producing a starter culture for cheese ripening, which method
comprises:
(i) providing samples of a ripening cheese over different time points during the ripening process of the cheese;
(ii) performing sensory analyses on the samples to determine the intensity of at least one attribute;
(iii) quantifying the lactic acid bacteria which are present in the samples;
(iv) correlating the intensity of at least one attribute with the quantified lactic acid bacteria; and
(v) combining different species of lactic acid bacteria to produce the starter culture.

3. Method according to claim 1 or claim 2, wherein providing samples of a ripening cheese over different time points during the ripening process of the cheese comprises providing samples at time points having an intermediate period of at least 2 weeks.

4. Method according to any of the claims 1 to 3, wherein the at least one attribute is an odour attribute chosen from the group consisting of intensity, butter, soil, fruity, prickly, sour, sweat, fatty and off odour.

5. Method according to any of the claims 1 to 4, wherein the at least one attribute is a flavor attribute chosen from the group consisting of intensity, salt, sour, sweet, dairy, butter, savory, butyric acid, fruity, nutty, off taste, feeling in the mouth, rubbery, hardness, sticky, crumbly, dry, mealy, crystals and fatty.

6. Method according to any of the claims 1 to 5, wherein the cheese is a cheese chosen from the group consisting of Gouda, Edam, Parmesan, Swiss, Cheddar, Gruyere, Camembert, Munster, Livanot, Torre de Savoie and Mimolette.

7. Method according to any of the claims 1 to 6, wherein quantifying the lactic acid bacteria which are present in the samples comprises a quantitative polymerase chain reaction.

8. Method according to any of the claims 1 to 7, wherein quantifying the lactic acid bacteria which are present in the samples comprises quantifying the different species of lactic acid bacteria.

9. Method according to any of the claims 1 to 8, wherein quantifying the lactic acid bacteria which are present in the samples comprises quantifying the living lactic acid bacteria and/or quantifying the dead lactic acid bacteria.

10. Method according to any of the claims 1 to 9, wherein correlating the intensity of at least one attribute with the quantified lactid acid bacteria is carried out by correlation analysis, preferably a principal component analysis and/or an univariate correlation analysis.

11. Method according to any of the claims 2 to 10, wherein combining different species of lactic acid bacteria to produce the starter culture comprises using the correlation of the intensity of at least one attribute with the quantified lactid acid bacteria to determine the amount of species of lactic acid bacteria in the starter culture.

12. Method according to any of the claims 1 to 11, wherein the lactic acid bacteria are chosen from the group consisting of *Streptococcus thermophilus, Lactococcus lactis* spp. *cremoris, Lactococcus lactis* spp. *lactis, Lactobacillus casei, Lactobacillus helveticus, Lactobacillus rhamnosus* and *Leuconostoc* species.

13. Gouda cheese comprising an amount of *Streptococcus thermophilus* cells of more than 8.32 * 10⁸ cells / gram cheese after 6 weeks of ripening, an amount of *Streptococcus thermophilus* cells of more than 4.03 * 10⁸ cells / gram cheese after 12 weeks of ripening and / or an amount of *Streptococcus thermophilus* cells of more than 8.49 * 10⁸cells / gram cheese after 24 weeks of ripening.

14. Gouda cheese comprising an amount of *Streptococcus thermophilus* cells of more than 1.90 * 10⁹ cells / gram cheese after 6 weeks of ripening, an amount of *Streptococcus thermophilus* cells of more than 1.80 * 10⁹ cells / gram cheese after 12 weeks of ripening and / or an amount of *Streptococcus thermophilus* cells of more than 1.90 * 10⁹ cells / gram cheese after 24 weeks of ripening.


SEQUENCE LISTING

<110> DSM IP Assets B.V.

<120> CHEESE RIPENING

<130> 32067-EP-EPA

<160> 16

<170> BiSSAP 1.2

<210> 1
<211> 21
<212> DNA
<213> Artificial Sequence

<220>
<221> source
<222> 1..21
<223> /organism="Artificial Sequence"
      /note="L. lactis ssp. cremoris"
      /mol_type="unassigned DNA"

<400> 1
tcctgattca cagtaaatcc t                                                21


<210> 2
<211> 21
<212> DNA
<213> Artificial Sequence

<220>
<221> source
<222> 1..21
<223> /organism="Artificial Sequence"
      /note="L. lactis ssp. cremoris"
      /mol_type="unassigned DNA"

<400> 2
gcgcacttga atcagtttca a                                                21


<210> 3
<211> 21
<212> DNA
<213> Artificial Sequence

<220>
<221> source
<222> 1..21
<223> /organism="Artificial Sequence"
      /note="L. lactis ssp. lactis"
      /mol_type="unassigned DNA"

<400> 3
tcctgattca cagtaaatcc t                                                21


<210> 4
<211> 21
<212> DNA

<213> Artificial Sequence

<220>
<221> source
<222> 1..21
<223> /organism="Artificial Sequence"
      /note="L. lactis ssp. lactis"
      /mol_type="unassigned DNA"

<400> 4
atgccctcga atctgtttca a                                                21


<210> 5
<211> 18
<212> DNA
<213> Artificial Sequence

<220>
<221> source
<222> 1..18
<223> /organism="Artificial Sequence"
      /note="Lb. casei"
      /mol_type="unassigned DNA"

<400> 5
caccgagatt caacatgg                                                    18


<210> 6
<211> 19
<212> DNA
<213> Artificial Sequence

<220>
<221> source
<222> 1..19
<223> /organism="Artificial Sequence"
      /note="Lb. casei"
      /mol_type="unassigned DNA"

<400> 6
   cgatgcgaat ttctttttc                                                    19


<210> 7
<211> 21
<212> DNA
<213> Artificial Sequence

<220>
<221> source
<222> 1..21
<223> /organism="Artificial Sequence"
      /note="Lb. casei"
      /mol_type="unassigned DNA"

<400> 7
acaagctatg aattcacttg c                                                21


<210> 8
<211> 22

<212> DNA
<213> Artificial Sequence

<220>
<221> source
<222> 1..22
<223> /organism="Artificial Sequence"
      /note="S. thermophilus"
      /mol_type="unassigned DNA"

<400> 8
ttatttgaaa ggggcaattg ct                                              22


<210> 9
<211> 21
<212> DNA
<213> Artificial Sequence

<220>
<221> source
<222> 1..21
<223> /organism="Artificial Sequence"
      /note="S. thermophilus"
      /mol_type="unassigned DNA"

<400> 9
gtgaactttc cactctcaca c                                               21


<210> 10
<211> 24
<212> DNA
<213> Artificial Sequence

<220>
<221> source
<222> 1..24
<223> /organism="Artificial Sequence"
      /note="Lb. helveticus"
      /mol_type="unassigned DNA"

<400> 10
ttgatggtga agacttgctt agaa                                            24


<210> 11
<211> 21
<212> DNA
<213> Artificial Sequence

<220>
<221> source
<222> 1..21
<223> /organism="Artificial Sequence"
      /note="Lb. helveticus"
      /mol_type="unassigned DNA"

<400> 11
aggtcccact ggttcggtct c                                               21


<210> 12

<211> 20
<212> DNA
<213> Artificial Sequence

<220>
<221> source
<222> 1..20
<223> /organism="Artificial Sequence"
      /note="Lb. rhamnosus"
      /mol_type="unassigned DNA"

<400> 12
ccgatgaaca ggctcagtga                                                   20


<210> 13
<211> 20
<212> DNA
<213> Artificial Sequence

<220>
<221> source
<222> 1..20
<223> /organism="Artificial Sequence"
      /note="Lb. rhamnosus"
      /mol_type="unassigned DNA"

<400> 13
catgttgtgc gcttggaaaa                                                   20


<210> 14
<211> 18
<212> DNA
<213> Artificial Sequence

<220>
<221> source
<222> 1..18
<223> /organism="Artificial Sequence"
      /note="Lb. rhamnosus"
      /mol_type="unassigned DNA"

<400> 14
ttgcacttga ttgtttcg                                                     18


<210> 15
<211> 22
<212> DNA
<213> Artificial Sequence

<220>
<221> source
<222> 1..22
<223> /organism="Artificial Sequence"
      /note="Leuconostoc"
      /mol_type="unassigned DNA"

<400> 15
caggtcttga catcctttga ag                                                22

<210> 16
<211> 15
<212> DNA
<213> Artificial Sequence

<220>
<221> source
<222> 1..15
<223> /organism="Artificial Sequence"
      /note="Leuconostoc"
      /mol_type="unassigned DNA"

<400> 16
cgctcgttgc gggac                                                        15